# EUROPEAN PATENT APPLICATION

(11) **EP 3 984 595 A1**
(43) Date of publication of application: **20.04.2022**
(21) Application number: 20822961.7
(22) Date of filing: 10.06.2020
(51) Int. Cl.: A61P 1/00, A61P 3/00, A61P 3/10, A61P 9/00, A61P 25/00, A61P 25/24, A61P 25/28, A61P 29/00, A61P 31/00, C07K 7/06, A61K 38/08, C12P 21/06

(54) **PEPTIDE AND USE THEREOF**

(30) Priority: 11.06.2019 JP 2019109089
(71) Applicant: AJINOMOTO CO., INC., Chuo-ku Tokyo 104-8315 (JP)
(72) Inventor: KITAHARA, Yoshiro, Kawasaki-shi, Kanagawa 210-8681 (JP); OKAMATSU, Yoriko, Kawasaki-shi, Kanagawa 210-8681 (JP); SHIMBO, Kazutaka, Kawasaki-shi, Kanagawa 210-8681 (JP); ARASHIDA, Naoko, Kawasaki-shi, Kanagawa 210-8681 (JP); SHIRASAWA, Ayaka, Kawasaki-shi, Kanagawa 210-8681 (JP)
(74) Representative: Strehl Schübel-Hopf & Partner
(86) International application number: PCT/JP2020/022817
(87) International publication number: WO 2020/250923

(57) **Abstract**

The present invention aims to provide a novel peptide, a novel use of a peptide and a production method of the peptide.

An agent for improving intestinal barrier function, an agent for suppressing blood glucose elevation, an agent for improving insulin sensitivity, an agent for promoting FGF21 secretion, an agent for suppressing stress or protecting nerves, or an agent for reducing fatigue, each containing a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6). A peptide consisting of the amino acid sequence of any of SEQ ID NO: 1, SEQ ID NO: 4, SEQ ID NO: 5, and SEQ ID NO: 6. A method for producing a hydrolysate of a whey protein containing a peptide consisting of the amino acid sequence of any of SEQ ID NO: 1 - SEQ ID NO: 6, including a step of hydrolyzing the whey protein with chymotrypsin.

## Description

### [Technical Field]

The present invention relates to a novel peptide and a novel use of a peptide. In addition, the present invention relates to a production method of the peptide.

### [Background Art]

Patent document 1 and non-patent document 1 describe that a peptide having the amino acid sequence LIVTQTMKG (SEQ ID NO: 7) at the N-terminal has a ghrelin secretion suppressive action, and also an appetite suppressive action based on the suppression of ghrelin secretion. However, these documents do not describe an intestinal barrier function improving action, a blood glucose elevation suppressive action, an insulin sensitivity improving action, an FGF21 (fibroblast growth factor 21) secretion promoting action, a stress suppressive action, a neuroprotective action, or a fatigue reducing action.

Non-patent documents 2 and 3 show that ghrelin improves the intestinal barrier function. Non-patent document 4 describes that ghrelin improves insulin sensitivity based on the meta-analysis of the studies made in the past. Therefore, the intestinal barrier function improving action, insulin sensitivity improving action, and blood glucose level elevation suppressive action cannot be assumed from the ghrelin secretion suppressive action described in patent document 1 and non-patent document 1.

In addition, there is no known document or the like showing the relationship between ghrelin and FGF21.

### [Document List]

### [Patent document]

patent document 1: WO 2017/150536

### [Non-patent documents]

non-patent document 1: H. Aoki et al., "Lacto-ghrestatin, a novel bovine milk-derived peptide, suppresses ghrelin secretion", FEBS Letters 591 (2017) 2121-2130
non-patent document 2: R. Wu et al., "Orexigenic Hormone Ghrelin Ameliorates Gut Barrier Dysfunction In Sepsis In Rats", Critical Care Medicine, 2009 August; 37(8): 2421-2426; doi: 10.1097/CCM.0b013e3181a557a2
non-patent document 3: Y. Cheng et al., "Ghrelin Attenuates Intestinal Barrier Dysfunction Following Intracerebral Hemorrhage in Mice", International Journal of Molecular Sciences, 2016, 17, 2032; doi: 10.3390/ijms17122032
non-patent document 4: C. Zhang et al., "The Correlation Between Circulating Ghrelin and Insulin Resistance in Obesity: A Meta-Analysis", Frontiers in Physiology, September 2018, Volume 9, Article 1308; doi: 10.3389/fphys.2018.01308

### [Summary of Invention]

### [Technical Problem]

The present invention aims to provide a novel peptide and a novel use of peptide. In addition, the present invention aims to provide a production method of the peptide.

### [Solution to Problem]

The present inventors analyzed the gastrointestinal contents after whey (whey protein) administration to gastrointestinal bypass surgery model animals, and found for the first time that a peptide consisting of a specific amino acid sequence has an intestinal barrier function improving action, a blood glucose elevation suppressive action, an insulin sensitivity improving action, and an FGF21 secretion promoting action. The present inventors have further found for the first time that the peptide consisting of the specific amino acid sequence has a stress suppressive action, a neuroprotective action, and a fatigue reducing action. Based on these new findings, the present inventors further conducted intensive studies and completed the present invention.

That is, the present invention provides the following.
[1] An agent for improving the intestinal barrier function, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6) (hereinafter these are sometimes to be collectively referred to as "the peptide relating to the present invention").
[2] An agent for suppressing blood glucose elevation, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).
[3] An agent for improving insulin sensitivity, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).
[4] An agent for promoting FGF21 secretion, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).
[5] An agent for suppressing stress or protecting nerves, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).
[6] An agent for reducing fatigue, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).
[7] A peptide consisting of the amino acid sequence of any of LIVTQTMKGL (SEQ ID NO: 1), IVTQTMKGL (SEQ ID NO: 4), IVTQTMKG (SEQ ID NO: 5), and VTQTMKGL (SEQ ID NO: 6).
[8] A method for producing a hydrolysate of a whey protein comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), VTQTMKGL (SEQ ID NO: 6), comprising a step of hydrolyzing the whey protein with chymotrypsin.

### [Advantageous Effects of Invention]

The peptide relating to the present invention has an intestinal barrier function improving action, a blood glucose elevation suppressive action, an insulin sensitivity improving action, an FGF21 secretion promoting action, a stress suppressive action, a neuroprotective action, or a fatigue reducing action, and can be used as a medicament, a food, or the like for use based on the action.

### [Brief Description of Drawings]

Fig. 1 shows blood FD-4 concentration (Mean±SEM (n=5)) after oral administration of compound 1 (peptide consisting of the amino acid sequence of LIVTQTMKGL (SEQ ID NO: 1)) in Experimental Example 1.
Fig. 2 shows ΔAUC (Mean±SEM (n=6 - 7)) at the time when an oral glucose tolerance test (OGTT) was performed in Experimental Example 2.
Fig. 3 shows the transition from the blood glucose level at 0 min when an insulin tolerance test (ITT) was performed in Experimental Example 3 (Fig. 3(a)) and ΔAUC (Fig. 3(b)) (Mean±SEM (n=4 - 6)).
Fig. 4 shows blood FGF21 concentration (Mean±SEM (n=5)) in Experimental Example 4.
Fig. 5 shows blood acetyl-L-carnitine concentration (Mean±SEM (n=3)) in Experimental Example 5.
Fig. 6 shows blood ornithine/citrulline ratio (Mean±SEM (n=5)) in Experimental Example 6.
Fig. 7 shows concentration (Mean±SEM (n=2)) of compound 1 in Production Example 7.

### [Description of Embodiments]

The peptide relating to the present invention includes the following (1) - (6).
(1) a peptide consisting of the amino acid sequence of LIVTQTMKGL (SEQ ID NO: 1)
(2) a peptide consisting of the amino acid sequence of LIVTQTMKG (SEQ ID NO: 2)
(3) a peptide consisting of the amino acid sequence of LIVTQTMK (SEQ ID NO: 3)
(4) a peptide consisting of the amino acid sequence of IVTQTMKGL (SEQ ID NO: 4)
(5) a peptide consisting of the amino acid sequence of IVTQTMKG (SEQ ID NO: 5)
(6) a peptide consisting of the amino acid sequence of VTQTMKGL (SEQ ID NO: 6)

As described above, the peptide relating to the present invention has an intestinal barrier function improving action, a blood glucose elevation suppressive action, an insulin sensitivity improving action, an FGF21 secretion promoting action, a stress suppressive action, a neuroprotective action, and a fatigue reducing action. In the below-mentioned agent of the present invention, only one kind of the peptide relating to the present invention may be used, or two or more kinds may be used in combination.

The peptide relating to the present invention can be used not only in a free form but also in the form of a salt, hydrate, or solvate. The term "peptide" in the present specification is a concept also encompassing salt, hydrate, and solvate. The salt form of the peptide relating to the present invention is, for example, a salt acceptable as a medicament or food. Examples thereof include acid addition salts (e.g., inorganic acid salts such as hydrochloride, sulfate, nitrate, phosphate and the like, organic acid salts such as acetate, maleate, fumarate, citrate, malate, lactate, α-ketoglutarate, gluconate, caprylate and the like), metal salts (e.g., alkali metal salts such as sodium salt, potassium salt and the like, alkaline earth metal salts such as magnesium salt, calcium salt and the like, aluminum salt, zinc salt), ammonium salts (e.g., salts with ammonium, tetramethylammonium, etc.), and the like.

In the present invention, the amino acid constituting the peptide may be an L-form or a D-form.

The peptide relating to the present invention can be produced, for example, by a solid-phase synthesis method and the like shown below.

As the carrier to be used for solid-phase synthesis, a carrier capable of binding to the C-terminal carboxyl group of the peptide chain via a linker is generally used for the resin. Representative examples of such solid-phase carrier include Wang resin, AM resin, TGR resin and the like.

The amino acid to be used for solid-phase synthesis is preferably one in which the amino group of the main chain is protected by a 9-fluorenylmethylcarbonyl (Fmoc) group or a t-butoxycarbonyl (Boc) group, though it is not limited to these. When a hydroxyl group, a thiol group, an amino group, a carboxyl group or the like is present in the side chain of amino acid, these functional groups are preferably protected by a protecting group other than Fmoc group and Boc group.

The protective amino acid can be introduced into the carrier by a known method. For example, a method using a carbodiimide-based condensing agent as the condensing agent can be mentioned. Examples of the aforementioned carbodiimide-based condensing agent include dicyclohexylcarbodiimide (DCC), diisopropylcarbodiimide (DIPC), 1-ethyl-3-(3-dimethylaminopropyl)carbodiimide hydrochloride (WSCI) and the like. As the solvent used in the reaction, DCM, tetrahydrofuran, toluene and the like can be used. The reaction is preferably performed at room temperature.

The Fmoc group can be removed by adding a secondary amine to the protected amino acid-carrier obtained above. As the aforementioned reaction solvent, dimethylformamide (DMF) is preferably used. As the aforementioned secondary amine, piperidine is generally used, and pyrrolidine, diethylamine, dibutylamine, diisopropylamine and the like can also be used. The above-mentioned reaction can be performed at a reaction temperature of from 0°C to the boiling point of the solvent, and the reaction is preferably performed at room temperature. The carrier after the reaction can be taken out from the solvent by filtration or the like.

The carrier into which the amino acid after removal of Fmoc obtained above has been introduced is swollen again in DMF, and the protected amino acid is reacted. As the condensing agent, dicyclohexylcarbodiimide (DCC), diisopropylcarbodiimide (DIPC), 1-ethyl-3-(3-dimethylaminopropyl)carbodiimide hydrochloride (WSCI), 1-[bis (dimethylamino)methylene]-1H-1,2,3-triazolo [4,5-b]pyridinium 3-oxide hexafluorophosphate (HATU), 1-hydroxybenzotriazole (HOBt), 1-hydroxy-7-azabenzotriazole (HOAt) and the like can be used alone or a mixture thereof can be used. The above-mentioned reaction can be performed at a reaction temperature from 0°C to the boiling point of the solvent, and the reaction is preferably performed at room temperature. The elongation of the peptide chain can be confirmed by the Kaiser test, and the carrier after the reaction can be taken out from the solvent by filtration and the like.

The peptide can be cut out from the carrier by a known method. For example, the peptide is cut out using a strong acid such as trifluoroacetic acid and the like. At this time, the protecting group of the side chain of each amino acid in the peptide may be removed simultaneously.

In addition, the peptide relating to the present invention can also be produced by allowing a hydrolysis enzyme to act on whey protein. The protein hydrolysis enzyme to be used for hydrolyzing whey protein is not particularly limited, but an enzyme having a protease activity or peptidase activity and usable for food production is preferred. As such enzyme, for example, chymotrypsin can be mentioned.

In the production method, as the whey protein to be the substrate for enzymatic reactions, for example, purified milk β-lactoglobulin can be mentioned. It is not limited thereto, and milk or whey containing whey protein may be used as it is as a substrate.

In the production method, the amount of the protein hydrolysis enzyme to be used is, for example, an amount that renders the mass ratio of protein hydrolysis enzyme and substrate (whey protein) (protein hydrolysis enzyme:substrate) 1:20 - 1:1000.

The enzyme reaction time is, for example, 30 min - 24 hr, preferably about 2 hr - 8 hr. The enzyme reaction temperature is, for example, 25 - 70°C, preferably 37°C. The enzyme reaction is performed at, for example, pH 5 - 9, preferably pH 6 - 8.

After completion of the enzyme reaction, the enzyme is deactivated as appropriate, and a hydrolysate of a whey protein containing the peptide relating to the present invention can be obtained. The obtained hydrolysate can be used as it is as the agent of the present invention described later, or may be separated and purified by a known method to give the peptide relating to the present invention.

In one embodiment, the present invention relates to an agent for improving intestinal barrier function, containing the peptide relating to the present invention.

In the present invention, the "intestinal barrier function" is a function to prevent the invasion of microorganisms into the intestinal tissue by the physical wall of intestinal epithelial cell, mucous layer, sugar coating, and the like, the secretion of molecules having antibacterial activity, and the like.

In the present invention, the intestinal barrier function improving action can be evaluated, for example, by the method of the below-mentioned Experimental Example 1 or a method analogous thereto.

Based on the intestinal barrier function improving action, the agent for improving intestinal barrier function of the present invention is expected to be usable for the prophylaxis or treatment (improvement) of metabolic diseases, intestinal infections, cognitive functional decline, depression, stress, inflammatory diseases, age-related symptoms, and cardiovascular diseases, life extension, and health maintenance.

In one embodiment, the present invention relates to an agent for suppressing blood glucose elevation, containing the peptide relating to the present invention.

In the present invention, the "blood glucose elevation" means an increase in blood glucose level caused by meal intake, and generally means an increase in blood glucose level that occurs within about 3 to 5 hours after eating.

In the present invention, the blood glucose elevation suppressive action can be evaluated, for example, by the method of the below-mentioned Experimental Example 2 or a method analogous thereto.

Based on the blood glucose elevation suppressive action, the agent for suppressing blood glucose elevation of the present invention is expected to be usable for the prophylaxis or treatment (improvement) of metabolic diseases, cognitive functional decline, depression, stress, inflammatory diseases, age-related symptoms, and cardiovascular diseases, life extension, and health maintenance.

In one embodiment, the present invention relates to an agent for improving insulin sensitivity, containing the peptide relating to the present invention.

In the present invention, the "insulin sensitivity" refers to the easiness of action for insulin in the body. When insulin sensitivity is high, insulin can exert its action sufficiently, and when insulin sensitivity is low, insulin cannot exert its action sufficiently. The action of insulin refers to the action of regulating glucose/lipid/protein metabolism, the action of inducing cell proliferation and cell differentiation, and the like.

In the present invention, the insulin sensitivity improving action can be evaluated, for example, by the method of the below-mentioned Experimental Example 3 or a method analogous thereto.

Based on the insulin sensitivity improving action, the agent for improving insulin sensitivity of the present invention is expected to be usable for the prophylaxis or treatment (improvement) of metabolic diseases, cognitive functional decline, depression, stress, inflammatory diseases, age-related symptoms, and cardiovascular diseases, life extension, and health maintenance.

In one embodiment, the present invention relates to an agent for promoting FGF21 secretion, containing the peptide relating to the present invention.

FGF21 is an intercellular signal factor mainly produced in the liver and the like, and is involved in the regulation of proliferation, differentiation and metabolism of various cells.

In the present invention, the FGF21 secretion promoting action can be evaluated, for example, by the method of the below-mentioned Experimental Example 4 or a method analogous thereto.

Based on the FGF21 secretion promoting action, the agent for promoting FGF21 secretion of the present invention is expected to be usable for the prophylaxis or treatment (improvement) of metabolic diseases, cognitive functional decline, depression, stress, inflammatory diseases, age-related symptoms, and cardiovascular diseases, life extension, and health maintenance.

Many reports have been made on the relationship between FGF21 and metabolic diseases, cognitive functional decline, depression, stress, inflammatory diseases, and cardiovascular diseases (e.g., EMBO Molecular Medicine (2018) 10, e8791; Hormones and Behavior 85 (2016) 86-95; Psychiatry Research 252 (2017) 111-113; Molecular Psychiatry (2015) 20, 215-223; Endocrinology, June 2012, 153(6), 2689-2700; Cellular Signalling 40 (2017) 10-21; Reviews in Endocrine and Metabolic Disorders, https://doi.org/10.1007/s11154-019-09488-x).

In one embodiment, the present invention relates to an agent for suppressing stress or protecting nerves, containing the peptide relating to the present invention.

In the present invention, the "suppression of stress" refers to suppressing the psychological, physical, and behavioral effects caused by "physical stressor" (heat and cold, noise and congestion, and the like), "chemical stressor" (pollutant, drug, oxygen deficiency/excess, carbon monoxide, and the like), and "psychological/social stressor" (human relations, work problems, family problems, and the like).

In the present invention, the "neuroprotection" refers to the protection of central nerve and peripheral nerve from losing function due to physical and chemical factors.

In the present invention, the stress suppressive action and the neuroprotective action can be evaluated, for example, by the method of the below-mentioned Experimental Example 5 or a method analogous thereto.

Based on the stress suppressive action or neuroprotective action, the agent for suppressing stress or protecting nerves of the present invention is expected to be usable for the prophylaxis or treatment (improvement) of cognitive functional decline, depression, stress, inflammatory diseases, age-related symptoms, and cardiovascular diseases, life extension, and health maintenance.

In one embodiment, the present invention relates to an agent for reducing fatigue, containing the peptide relating to the present invention.

In the present invention, "reducing fatigue" refers to reducing fatigue, decreased motivation, decreased concentration, and the like that are caused by accumulation of physical and mental loads.

In the present invention, the fatigue reducing action can be evaluated, for example, by the method of the below-mentioned Experimental Example 6 or a method analogous thereto.

Based on the fatigue reducing action, the agent for reducing fatigue of the present invention is expected to be usable for the prophylaxis or treatment (improvement) of cognitive functional decline, depression, stress, inflammatory diseases, age-related symptoms, and cardiovascular diseases, life extension, and health maintenance.

The agent for improving intestinal barrier function, the agent for suppressing blood glucose elevation, the agent for improving insulin sensitivity, the agent for promoting FGF21 secretion, the agent for suppressing stress or protecting nerves, and the agent for reducing fatigue of the present invention (hereinafter these are sometimes to be collectively referred to as "the agent of the present invention") may be the peptide relating to the present invention per se, or a composition containing the peptide relating to the present invention and other components (e.g., carrier acceptable as medicament or food) (e.g., pharmaceutical composition, food composition).

The "agent" in the present invention is a concept encompassing medicaments and foods.

The agent of the present invention can be safely administered orally or parenterally to subjects such as human, mammals (e.g., mouse, rat, hamster, rabbit, cat, dog, bovine, sheep, monkey), birds (e.g., chicken, turkey), and the like.

The form of the agent of the present invention is not particularly questioned and may be, for example, powder, granule, tablet, capsule, liquid (e.g., solution, suspension, emulsion), drink, jelly, pudding, yogurt, candy, chewing gum or the like. These can be produced by a known method. For example, the peptide relating to the present invention is mixed with carriers acceptable as food or medicament (e.g., excipient, binder, disintegrant, lubricant, solvent) and powder, granule, tablet, capsule, liquid and the like can be produced by a method known in the field of food preparation or pharmaceutical preparation. In addition, they can also be produced by adding and mixing the peptide relating to the present invention to and with food (e.g., general foods, drinks (e.g., water, soft drink)).

In the present specification, food is a concept that broadly encompasses foods that can be taken orally (excluding pharmaceuticals) and includes not only so-called "food" but also drink, health supplement, food with health claims (e.g., food for specified health uses, foods with functional claims, food with nutrient function claims), supplement and the like.

In the agent for improving intestinal barrier function of the present invention, the dose (amount of intake) of the peptide relating to the present invention is, for example, 0.05 mg - 500 g, preferably 0.5 mg - 50 g, more preferably 5 mg - 10 g, per day for an adult (body weight 60 kg).

In the agent for suppressing blood glucose elevation of the present invention, the dose (amount of intake) of the peptide relating to the present invention is, for example, 0.05 mg - 500 g, preferably 0.5 mg - 50 g, more preferably 5 mg - 10 g, per day for an adult (body weight 60 kg).

In the agent for improving insulin sensitivity of the present invention, the dose (amount of intake) of the peptide relating to the present invention is, for example, 0.05 mg - 500 g, preferably 0.5 mg - 50 g, more preferably 5 mg - 10 g, per day for an adult (body weight 60 kg).

In the agent for promoting FGF21 secretion of the present invention, the dose (amount of intake) of the peptide relating to the present invention is, for example, 0.05 mg - 500 g, preferably 0.5 mg - 50 g, more preferably 5 mg - 10 g, per day for an adult (body weight 60 kg).

In the agent for suppressing stress or protecting nerves of the present invention, the dose (amount of intake) of the peptide relating to the present invention is, for example, 0.05 mg - 500 g, preferably 0.5 mg - 50 g, more preferably 5 mg - 10 g, per day for an adult (body weight 60 kg).

In the agent for reducing fatigue of the present invention, the dose (amount of intake) of the peptide relating to the present invention is, for example, 0.05 mg - 500 g, preferably 0.5 mg - 50 g, more preferably 5 mg - 10 g, per day for an adult (body weight 60 kg).

In the agent of the present invention, the content of the peptide relating to the present invention can be appropriately selected from the amounts that make the dose (amount of intake) fall within the above-mentioned ranges.

The present invention also relates to a novel peptide consisting of the amino acid sequence of any of LIVTQTMKGL (SEQ ID NO: 1), IVTQTMKGL (SEQ ID NO: 4), IVTQTMKG (SEQ ID NO: 5), and VTQTMKGL (SEQ ID NO: 6). The definition and production method of each peptide are the same as those described above for the agent of the present invention. As described above, these peptides have an intestinal barrier function improving action, a blood glucose elevation suppressive action, an insulin sensitivity improving action, an FGF21 secretion promoting action, a stress suppressive action, a neuroprotective action, and a fatigue reducing action, and can be used as medicaments, foods and the like for use based on such actions.

### [Example]

The present invention is described more specifically in the following by referring to Production Examples and Experimental Examples; however, the present invention is not limited by these Production Examples and Experimental Examples.

In the following Production Examples and Experimental Examples, compounds 1 - 6 show the following.
compound 1: a peptide consisting of the amino acid sequence of LIVTQTMKGL (SEQ ID NO: 1)
compound 2: a peptide consisting of the amino acid sequence of LIVTQTMKG (SEQ ID NO: 2)
compound 3: a peptide consisting of the amino acid sequence of LIVTQTMK (SEQ ID NO: 3)
compound 4: a peptide consisting of the amino acid sequence of IVTQTMKGL (SEQ ID NO: 4)
compound 5: a peptide consisting of the amino acid sequence of IVTQTMKG (SEQ ID NO: 5)
compound 6: a peptide consisting of the amino acid sequence of VTQTMKGL (SEQ ID NO: 6)

### [Production Example 1] Production of compound 1

Using the Fmoc-Leu-Wang resin as a starting material, the peptide chain was extended by the 9-fluorenylmethoxycarbonyl method (Fmoc method) to synthesize the desired protected peptide resin. Then, it was treated with trifluoroacetic acid to remove the resin and perform deprotection to obtain a crude peptide. The obtained crude peptide was purified by high performance liquid chromatography (HPLC) and lyophilized to obtain the desired product as a white powder.
yield 3.0 g (purity 98.3%)
ESI-MS: MW=1103.0 (calculated 1103.4)
amino acid analytical value; Thr (2) 1.90, Glu (1) 1.01, Gly (1) 0.98, Val (1) 0.80, Met (1) 0.97, Ile (1) 0.79, Leu (2) 2.0, Lys (1) 1.01, NH3 (1) 1.10

### [Production Example 2] Production of compound 2

Using the Fmoc-Gly-Wang resin as a starting material, the peptide chain was extended by the 9-fluorenylmethoxycarbonyl method (Fmoc method) to synthesize the desired protected peptide resin. Then, it was treated with trifluoroacetic acid to remove the resin and perform deprotection to obtain a crude peptide. The obtained crude peptide was purified by high performance liquid chromatography (HPLC) and lyophilized to obtain the desired product as a white powder.
yield 1.26 g (purity 99.3%)
ESI-MS: MW=990.2 (calculated 990.2)
amino acid analytical value; Thr (2) 1.89, Glu (1) 1.00, Gly (1) 0.97, Val (1) 0.81, Met (1) 0.98, Ile (1) 0.80, Leu (1) 0.99, Lys (1) 1.01, NH3 (1) 1.10

### [Production Example 3] Production of compound 3

Using the Fmoc-Lys(Boc)-Wang resin as a starting material, the peptide chain was extended by the 9-fluorenylmethoxycarbonyl method (Fmoc method) to synthesize the desired protected peptide resin. Then, it was treated with trifluoroacetic acid to remove the resin and perform deprotection to obtain a crude peptide. The obtained crude peptide was purified by high performance liquid chromatography (HPLC) and lyophilized to obtain the desired product as a white powder.
yield 1.26 g (purity 99.0%)
ESI-MS: MW=933.1 (calculated 933.2)
amino acid analytical value; Thr (2) 1.89, Glu (1) 1.00, Val (1) 0.81, Met (1) 0.98, Ile (1) 0.80, Leu (1) 0.99, Lys (1) 1.01, NH3 (1) 1.10

### [Production Example 4] Production of compound 4

Using the Fmoc-Leu-Wang resin as a starting material, the peptide chain was extended by the 9-fluorenylmethoxycarbonyl method (Fmoc method) to synthesize the desired protected peptide resin. Then, it was treated with trifluoroacetic acid to remove the resin and perform deprotection to obtain a crude peptide. The obtained crude peptide was purified by high performance liquid chromatography (HPLC) and lyophilized to obtain the desired product as a white powder.
yield 1.26 g (purity 99.4%)
ESI-MS: MW=990.0 (calculated 990.2)
amino acid analytical value; Thr (2) 1.89, Glu (1) 1.00, Gly (1) 0.99, Val (1) 0.68, Met (1) 0.98, Ile (1) 0.67, Leu (1) 1.00, Lys (1) 1.02, NH3 (1) 1.12

### [Production Example 5] Production of compound 5

Using the Fmoc-Gly-Wang resin as a starting material, the peptide chain was extended by the 9-fluorenylmethoxycarbonyl method (Fmoc method) to synthesize the desired protected peptide resin. Then, it was treated with trifluoroacetic acid to remove the resin and perform deprotection to obtain a crude peptide. The obtained crude peptide was purified by high performance liquid chromatography (HPLC) and lyophilized to obtain the desired product as a white powder.
yield 1.26 g (purity 99.3%)
ESI-MS: MW=876.9 (Calculated 877.1)
amino acid analytical value; Thr (2) 1.88, Glu (1) 1.00, Gly (1) 0.97, Val (1) 0.66, Met (1) 0.99, Ile (1) 0.65, Leu (1) 1.00, Lys (1) 1.01, NH3 (1) 1.09

### [Production Example 6] Production of compound 6

Using the Fmoc-Leu-Wang resin as a starting material, the peptide chain was extended by the 9-fluorenylmethoxycarbonyl method (Fmoc method) to synthesize the desired protected peptide resin. Then, it was treated with trifluoroacetic acid to remove the resin and perform deprotection to obtain a crude peptide. The obtained crude peptide was purified by high performance liquid chromatography (HPLC) and lyophilized to obtain the desired product as a white powder.
yield 1.26 g (purity 99.5%)
ESI-MS: MW=877.0 (Calculated 877.1)
amino acid analytical value; Thr (2) 1.87, Glu (1) 1.00, Gly (1) 0.98, Val (1) 1.00, Met (1) 0.99, Ile (1) 1.00, Leu (1) 1.00, Lys (1) 1.01, NH3 (1) 1.33

### [Experimental Example 1] Intestine permeability

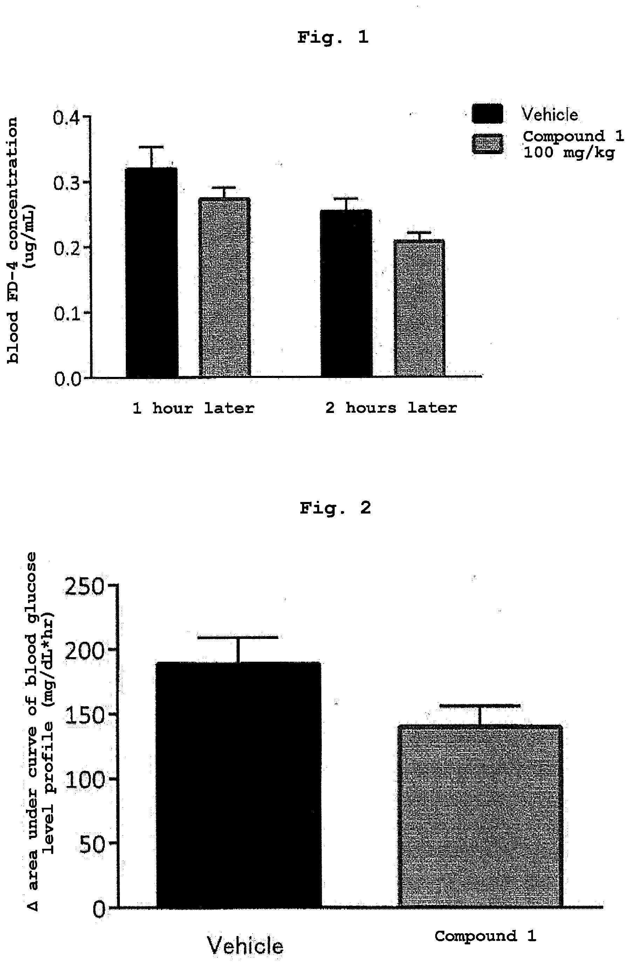

The administration medium (0.5% methylcellulose; vehicle) or compound 1 (100 mg/kg) was orally administered to male KK-Ay mice (11 weeks old) that had been fasted from 10 o'clock the day before, and FITC-dextran (FD-4) (300 mg/kg) was orally administered 1 hr later. Blood was collected from the tail vein 1 hr and 2 hr after FD-4 administration, and the plasma FD-4 concentration was measured. The results are shown in Fig. 1.

From Fig. 1, suppression of an increase in the blood FD-4 concentration was observed both 1 hr and 2 hr after oral administration of FD-4 in the compound 1 administration group as compared with the vehicle group. Thus, it was confirmed that compound 1 improves the intestinal barrier function.

### [Experimental Example 2] Oral glucose tolerance test (OGTT)

The administration medium (0.5% methylcellulose; vehicle) or compound 1 (30 mg/kg) was orally administered to male C57BL/6J mice (7 weeks old) that had been fasted from 17 o'clock the day before, and 2 g/kg of glucose was orally administered by gavage 1 hr later. Blood was collected from the tail vein before administration, and 15, 30, 60, 120 and 180 min after administration, and the blood glucose level was measured.

The blood glucose level after glucose loading remained low in the compound 1 administration group from 30 min after loading and thereafter. The results of the area under curve (ΔAUC) of the blood glucose level profile calculated with the 0-minute value as the standard are shown in Fig. 2.

From Fig. 2, the compound 1 administration group showed a low value as compared with the vehicle group, and a blood glucose elevation suppressive action of compound 1 was confirmed.

### [Experimental Example 3] Insulin tolerance test (ITT)

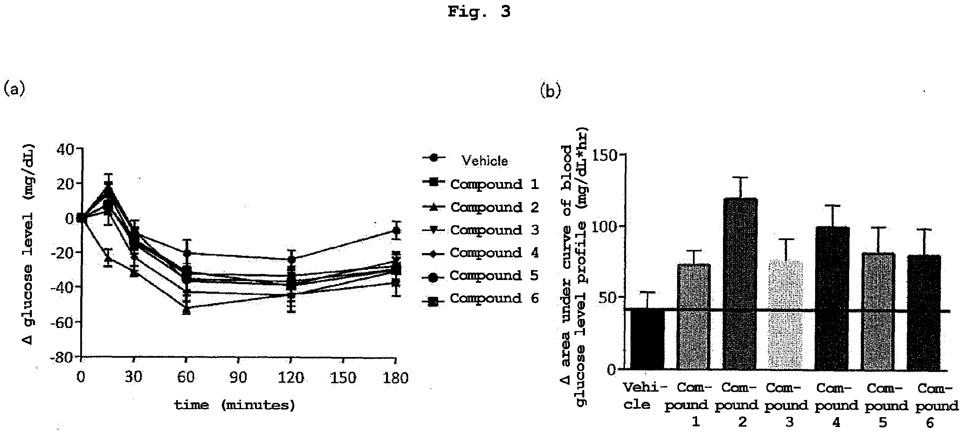

The administration medium (0.5% methylcellulose; vehicle) or compound 1 (30 mg/kg) was orally administered to male KK-Ay mice (7 - 12 weeks old) that had been fasted from 17 o'clock the day before, and insulin (0.5 U/kg) was subcutaneously administered 1 hr later. Blood was collected from the tail vein before administration, and 15, 30, 60, 120 and 180 min after administration, and the blood glucose level was measured. The blood glucose level was also measured for compounds 2 - 6 by a similar method. The profile from the blood glucose level at 0 min is shown in Fig. 3(a). The results of the area under curve (ΔAUC) of the blood glucose level profile calculated with the 0-minute value as the standard are shown in Fig. 3(b).

From Fig. 3(a) and (b), KK-Ay mouse had a strong insulin resistance, and the vehicle group did not show a clear decrease in the blood glucose level even when insulin was administered. However, compound 1 administration group showed a significant decrease in blood glucose. Such insulin sensitivity improving effect was similarly found in compounds 2 - 6.

### [Experimental Example 4] FGF21 secretion promoting action

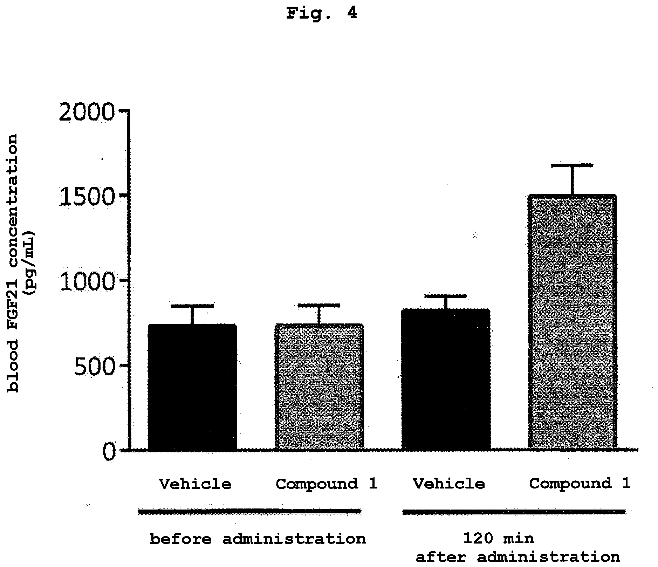

The administration medium (0.5% methylcellulose; vehicle) or compound 1 (30 mg/kg) was administered by gavage to male C57BL/6J mice (8 weeks old) that had been under fasting treatment for 6 hr. Blood was collected from the portal vein before administration and 120 min after administration, and the plasma FGF21 concentration was measured. The results are shown in Fig. 4.

From Fig. 4, an increase in the blood FGF21 concentration was confirmed 120 min after administration in the compound 1 administration group as compared with the vehicle group.

### [Experimental Example 5] Anti-stress, neuroprotective action

Male C57BL/6J mice (9-week-old) that had been under fasting treatment overnight were housed in a restraint stress cage for mice, and allowed to stand for 3 hr. Then, the administration medium (0.5% methylcellulose; vehicle) or compound 1 (30 mg/kg) was orally administered and blood was collected from the inferior vena cava 120 min after administration, and the plasma acetyl-L-carnitine concentration was measured. Acetyl-L-carnitine is an endogenous metabolite known to have an anti-stress and neuroprotective action (e.g., Nasca C et al., "L-acetylcarnitine causes rapid antidepressant effects through the epigenetic induction of mGlu2 receptors", Proc. Natl. Acad. Sci. USA, 2013, Mar 19; 110(12): 4804-4809; and Kazak F et al., "Neuroprotective effects of acetyl-l-carnitine on lipopolysaccharide-induced neuroinflammation in mice: Involvement of brain-derived neurotrophic factor", Neuroscience Letters, 2017 Sep 29; 658: 32-36).

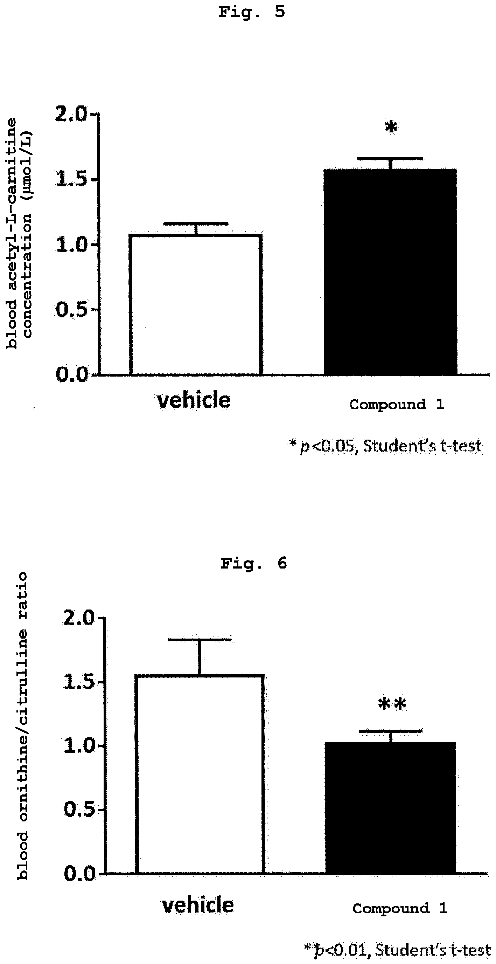

The results are shown in Fig. 5.

From Fig. 5, an increase in the blood acetyl-L-carnitine concentration was observed 120 min after administration in the compound 1 administration group as compared with the vehicle group, and a reaction to suppress stress or protect nerves was confirmed.

### [Experimental Example 6] Anti-fatigue action

The administration medium (0.5% methylcellulose; vehicle) or compound 1 (30 mg/kg) was administered by gavage to male C57BL/6J mice (7 weeks old) that had been under fasting treatment overnight. Blood was collected from the portal vein 120 min after administration, and the plasma ornithine and citrulline concentrations were measured. The ornithine/citrulline ratio in plasma is known as a biomarker that increases during fatigue such as chronic fatigue syndrome and the like (e.g., Yamano E et al., "Index markers of chronic fatigue syndrome with dysfunction of TCA and urea cycles", Scientific Reports, 2016 Oct 11; 6: 34990, doi: 10.1038/srep34990).

The results are shown in Fig. 6.

From Fig. 6, the blood ornithine/citrulline ratio decreased 120 min after administration in the compound 1 administration group as compared with the vehicle group, and a biological reaction that reduces fatigue was observed.

### [Production Example 7] Production of milk protein hydrolysate

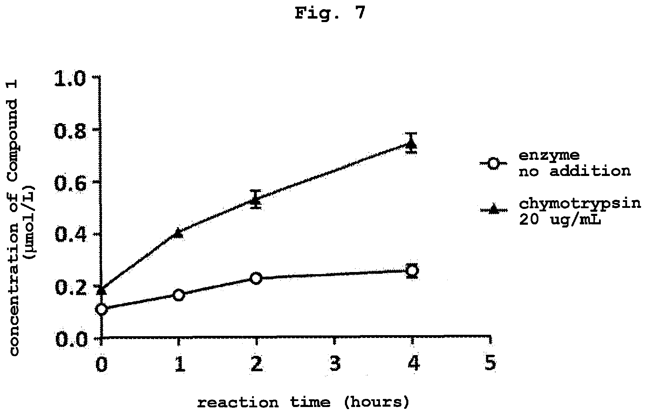

Purified milk β-lactoglobulin (final concentration 2 mg/mL) and chymotrypsin (final concentration 0.02 mg/mL) were mixed in a 10 mM phosphate buffer (pH 6.0) and reacted at 37°C. After the reaction was started, the reaction mixture was collected over time, and the concentration of compound 1 in the reaction mixture was measured by Q-TOF/MS. The results are shown in Fig. 7.

From Fig. 7, it was confirmed that compound 1 was produced in the reaction mixture in a reaction time-dependent manner. Therefore, it was confirmed that the compound can also be produced by enzymatic hydrolysis of milk protein.

### [Industrial Applicability]

The peptide relating to the present invention is useful as a medicament or food for the improvement of intestinal barrier function, suppression of blood glucose elevation, improvement of insulin sensitivity, promotion of FGF21 secretion, suppression of stress, neuroprotection, and reduction of fatigue.

This application is based on patent application No. 2019-109089 filed in Japan, the contents of which are encompassed in full herein.

## Claims

1. An agent for improving the intestinal barrier function, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).

2. An agent for suppressing blood glucose elevation, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).

3. An agent for improving insulin sensitivity, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).

4. An agent for promoting FGF21 secretion, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).

5. An agent for suppressing stress or protecting nerves, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).

6. An agent for reducing fatigue, comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), and VTQTMKGL (SEQ ID NO: 6).

7. A peptide consisting of the amino acid sequence of any of LIVTQTMKGL (SEQ ID NO: 1), IVTQTMKGL (SEQ ID NO: 4), IVTQTMKG (SEQ ID NO: 5), and VTQTMKGL (SEQ ID NO: 6).

8. A method for producing a hydrolysate of a whey protein comprising a peptide consisting of the amino acid sequence of any of LIVTQTMKGL (SEQ ID NO: 1), LIVTQTMKG (SEQ ID NO: 2), LIVTQTMK (SEQ ID NO: 3), IVTQTMKGL (SEQ ID NO: 4), IVTQTMKG (SEQ ID NO: 5), VTQTMKGL (SEQ ID NO: 6), comprising a step of hydrolyzing the whey protein with chymotrypsin.


SEQUENCE LISTING

<110>  AJINOMOTO CO., INC.

<120>  Peptide and Use Thereof

<130>  093023

<150>  JP 2019-109089
<151>  2019-06-11

<160>  7

<170>  PatentIn version 3.5

<210>  1
<211>  10
<212>  PRT
<213>  Artificial Sequence

<220>
<223>  Designed peptide

<400>  1

Leu Ile Val Thr Gln Thr Met Lys Gly Leu
1               5                   10


<210>  2
<211>  9
<212>  PRT
<213>  Artificial Sequence

<220>
<223>  Designed peptide

<400>  2

Leu Ile Val Thr Gln Thr Met Lys Gly
1               5


<210>  3
<211>  8
<212>  PRT
<213>  Artificial Sequence

<220>
<223>  Designed peptide

<400>  3

Leu Ile Val Thr Gln Thr Met Lys
1               5


<210>  4
<211>  9
<212>  PRT
<213>  Artificial Sequence

<220>

<223>  Designed peptide

<400>  4

Ile Val Thr Gln Thr Met Lys Gly Leu
1               5


<210>  5
<211>  8
<212>  PRT
<213>  Artificial Sequence

<220>
<223>  Designed peptide

<400>  5

Ile Val Thr Gln Thr Met Lys Gly
1               5


<210>  6
<211>  8
<212>  PRT
<213>  Artificial Sequence

<220>
<223>  Designed peptide

<400>  6

Val Thr Gln Thr Met Lys Gly Leu
1               5


<210>  7
<211>  9
<212>  PRT
<213>  Artificial Sequence

<220>
<223>  Designed peptide

<400>  7

Leu Ile Val Thr Gln Thr Met Lys Gly
1               5